# EUROPEAN PATENT APPLICATION

(11) **EP 4 721 814 A1**
(43) Date of publication of application: **08.04.2026**
(21) Application number: 25205646.0
(22) Date of filing: 30.09.2025
(51) Int. Cl.: A61N 5/10, G16H 20/40

(54) **NEURAL NETWORK TRAINING CORPUS DEVELOPMENT AND USE, AND USE OF A TRAINED NEURAL NETWORK RE RADIATION TREATMENT PLATFORM MACHINE CONTROL POINTS**

(30) Priority: 02.10.2024 US 202418904766
(71) Applicant: Siemens Healthineers International AG, 6312 Steinhausen (CH)
(72) Inventor: BASIRI, Shahab, 02570 Siuntio (FI); KUUSELA, Esa, 02320 Espoo (FI)
(74) Representative: Mathisen & Macara LLP

(57) **Abstract**

Information comprising physical settings for a particular radiation treatment platform (such as machine control points) form a training corpus. A neural network is trained 202 using that training corpus. By one approach, the training corpus does not include any information that pertains to any radiation treatment platform other than the particular radiation treatment platform. The training may be repeated as a function of at least one of a passage of time, a particular number of therapeutic uses of the particular radiation treatment platform, and/or completion of at least one maintenance activity. A radiation treatment plan can be optimized to provide optimized plan control points. These optimized plan control points can be mapped to corresponding machine control points as a function, at least in part, of the aforementioned trained neural network.

## Description

### TECHNICAL FIELD

These teachings relate generally to neural networks and also to treating a patient's planning target volume with energy pursuant to an optimized energy-based treatment plan.

### BACKGROUND

A neural network is a computational model inspired by the structure and functioning of the human brain, designed to recognize patterns and solve complex problems. It consists of interconnected units called neurons, which are organized in layers: an input layer that receives the data, one or more hidden layers that perform computations, and an output layer that delivers the final result. Each neuron applies a mathematical transformation to the input it receives and passes the result forward through the network. The strength of the connections between neurons, known as weights, are adjusted during a training process using a training corpus, allowing the network to learn by iteratively improving its predictions.

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

### SUMMARY

In a first aspect, the present invention provides a method as defined in claim 1. Optional features are specified in the dependent claims.

In a second aspect, the present invention provides apparatus as defined in claim 5. Optional features are specified in the dependent claims.

In a third aspect, the present invention provides a method as defined in claim 14. Optional features are specified in the dependent claims.

### BRIEF DESCRIPTION OF DRAWINGS

Various needs are at least partially met through provision of the neural network-based training corpus development, corresponding neural network training, and use with respect to radiation treatment platform machine control points described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings; and
FIG. 3 comprises a schematic representation as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

By one approach, an optimized radiation treatment plan can be at least partly defined by plan control points. Plan control points are often presented in DICOM format. DICOM refers to Digital Imaging and Communications in Medicine, and is a standard for storing, transmitting, retrieving, processing, printing, and displaying medical imaging information. DICOM files are represented by the extension .dcm and group information into data sets.

By one approach, the aforementioned plan control points are converted by the radiation treatment platform into timed trajectories or machine control points. Knowing machine control point parameters is typically important to estimating the delivery time, deciding the acceleration/deceleration of the radiation treatment platform mechanical axes, as well as adjusting Monitor Unit delivery rates.

Accurate estimation of the machine control point parameters is a challenging task that often relies on fast-response accurate machine emulators that take into account higher order gradients of all the moving axes. At least one problem in such regards is that the actual delivery of the optimized radiation treatment plan is subject to continuous monitoring of the performance of the machine during the delivery of the machine control points, and not all of the mechanical characteristics of the machine are typically taken into account during the creation of the machine control points. In addition, the foregoing is subject to change with the aging of the drive mechanisms and/or maintenance practices and events. As a result, an emulator may fail to correctly predict how the radiation treatment plan is actually performed. When the actual machine performance deviates from the expected, one or more of the relevant mechanical axes can deviate from a planned position. While these deviations are often monitored so as not to exceed set tolerances, such deviations can nevertheless contribute to inaccuracy of the treatment.

In some existing approaches, a machine controller uses linear approximators to estimate upcoming speed changes in an upcoming next few milliseconds during the treatment session (i.e., during delivery of the optimized radiation treatment plan by the radiation treatment platform). Unfortunately, linear modelling of such speed changes does not account for higher order derivates of the motion of the mechanical axes. The 3rd and 4th derivates, for example, can be important in these regards in addition to acceleration and speed. When an understanding of treatment delivery time is desired, that result is often calculated in the treatment planning system based on a simplified model and hence is subject to inherent error due to at least the foregoing issues with such a model.

Generally speaking, these teachings serve to at least partially address at least some of the foregoing issues. Pursuant to these various embodiments, a neural network training corpus can be developed and then utilized to train a neural network. Optimized plan control points can then be mapped to corresponding machine control points as a function, at least in part, of such a trained neural network.

By one approach, these teachings provide for accessing information comprising physical settings for a particular radiation treatment platform to form a training corpus. The aforementioned physical settings may comprise, for example, machine control points such as, but not limited to, gantry angles, a multi-leaf collimator position, a dose rate, and a patient support position.

A neural network can then be trained using that training corpus. By one approach, the training corpus does not include any information that pertains to any radiation treatment platform other than the particular radiation treatment platform. By one approach, the aforementioned neural network may comprise a recurrent neural network. The training itself may comprise, for example, supervised training. The training may be repeated as a function of at least one of a passage of time, a particular number of therapeutic uses of the particular radiation treatment platform, and/or completion of at least one maintenance activity.

By one approach, these teachings will accommodate optimizing a radiation treatment plan for a particular patient using the aforementioned particular radiation treatment platform, wherein the optimizing includes optimizing plan control points to provide optimized plan control points. These teachings will then accommodate mapping the optimized plan control points to corresponding machine control points as a function, at least in part, of the aforementioned trained neural network.

By one approach, these teachings will further accommodate outputting estimated machine control points from the trained neural network in advance of administering therapeutic radiation to a particular patient using the particular radiation treatment platform. In this case, and during administration of the therapeutic radiation to the particular patient during a treatment session, these teachings can provide for comparing the estimated machine control points to real-time physical parameter values of the particular radiation treatment platform and substituting at least some real-time physical parameter values for corresponding ones of the estimated machine control points upon detecting a triggering discrepancy while continuing administration of the therapeutic radiation to the particular patient.

So configured, these teachings can support, facilitate, and/or accomplish model-based prediction of actual treatment delivery parameters as well as provide an estimation of the treatment delivery time.

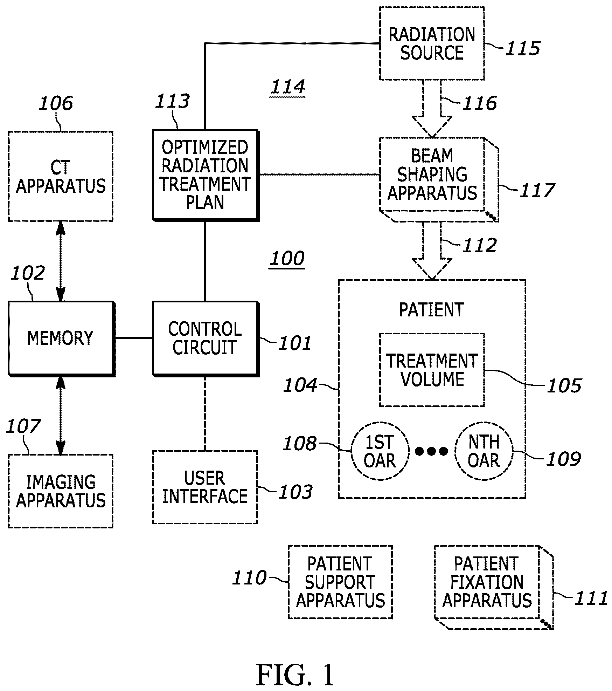

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

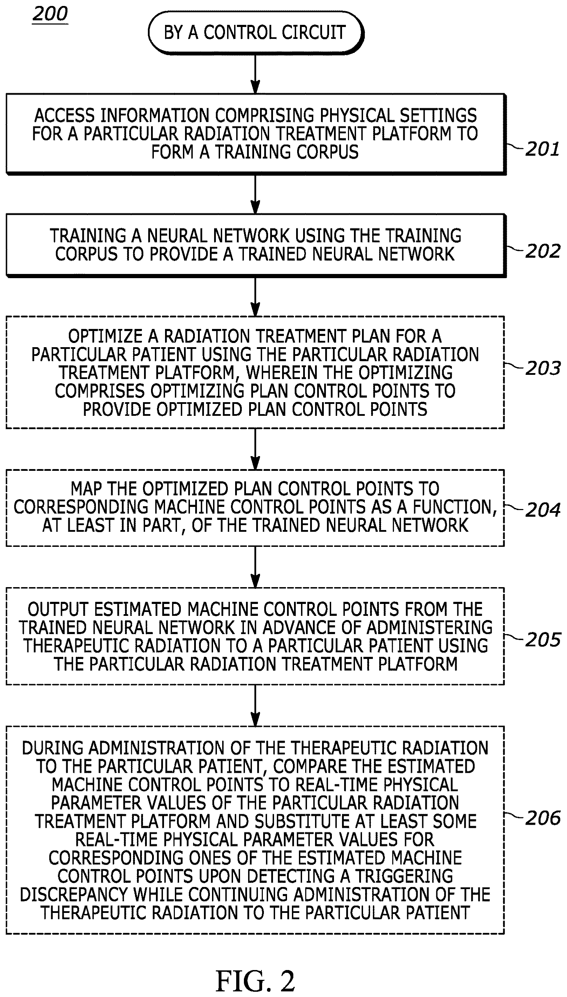

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for accessing information comprising physical settings for a particular radiation treatment platform to form a training corpus. These physical settings may comprise, for example, machine control points. Generally speaking, machine control points refer to specific parameters or settings on a radiation treatment platform that are adjusted to control the delivery of the prescribed radiation dose to a patient's tumor while minimizing exposure to surrounding healthy tissue. These machine control points include variables such as the beam energy, intensity, shape, and direction, which are configured based on the treatment plan developed for the patient. Examples of specific machine control points include the gantry angle, which determines the direction from which the radiation beam is delivered, multi-leaf collimator positions, which shape the beam to conform to the tumor's geometry and/or to modulate the beam, the dose rate, which affects the intensity of radiation delivered per unit time, and one or more patient support positions, which adjusts the patient's positioning relative to the beam. By precisely controlling one or more of these points, the radiation therapy machine can administer treatment according to complex plans, targeting the tumor with high accuracy while protecting healthy tissue.

By one approach, the accessed information comprises at least two of the foregoing illustrative machine control points. By one approach, these accessed physical settings comprise previous physical settings, such as previous machine control points, for the particular radiation treatment platform. By one approach, the resultant training corpus does not include any information that pertains to any radiation treatment platform other than the particular radiation treatment platform.

At block 202, the control circuit 101 trains a neural network (such as, for example, a recurrent neural network) using the aforementioned training corpus to thereby provide a trained neural network. This training may comprise, if desired, supervised training of the neural network. As an illustrative example in these regards, for some or all treated patients a clinic or other medical system may store previously optimized control points (OCP) as well as their corresponding machine control points (MCP) in DICOM format and in machine log files (using, for example, .txt format), respectively. In supervised learning, this historical data can be divided into two sets; a training set and a test set. A neural network can be trained over the training set to learn a map from the optimized control points to the machine control points (i.e., MCP = f(OCP)). Periodically, the quality of the map f(.) can be tested over the test set, which was not previously revealed to the neural network. The training can continue until the neural network can accurately estimate the machine control points from the given optimized control points in the test set.

In addition to initial training, these teachings will accommodate retraining the neural network as a function of at least one of a passage of time (such as a particular number of days or on a weekly or monthly basis), therapeutic uses of the particular radiation treatment platform (such as a particular number of therapeutic uses having occurred), and/or completion of at least one maintenance activity (such as replacement and/or adjustment or recalibration of one or more components of the radiation treatment platform such as one or more motors).

The end result of blocks 201 and 202 comprises a trained neural network that has been trained, in particular, with respect to physical settings such as machine control points for a particular radiation treatment platform.

At optional block 203, the control circuit 101 can then optimize a radiation treatment plan for a particular patient using the particular radiation treatment platform, wherein the optimizing comprises optimizing plan control points to provide optimized plan control points.

At optional block 204, the control circuit can then map those optimized plan control points to corresponding machine control points as a function, at least in part, of the trained neural network. In particular, with the plan control points as input, the trained neural network can output the corresponding machine control points.

At optional block 205, the control circuit 101 can output estimated machine control points from the trained neural network in advance of administering therapeutic radiation to that particular patient using the particular radiation treatment platform.

Then, at optional block 206, and during administration of the therapeutic radiation to the particular patient during a treatment session, the control circuit 101 can compare the aforementioned estimated machine control points to real-time physical parameter values of the particular radiation treatment platform and substitute at least some real- time physical parameter values for corresponding ones of the estimated machine control points upon detecting a triggering discrepancy (for example, when an estimated multi-leaf collimator leaf position deviates from the real-time position of the leaf) while continuing administration of the therapeutic radiation to the particular patient.

So configured, these teachings can employ a deep neural network model as a function approximator to map between the optimized plan control points and machine control points for a specific radiation treatment platform, so that the machine control points better (or best) match the actual machine performance.

The dimensionality of the problem is such that a fast-to-train simple recurrent neural network architecture may suffice in many application settings for accurate estimation. So configured, a treatment clinic can periodically (for example, monthly) retrain the model to ensure that the model accounts for changes in the mechanical characteristics of the radiation treatment platform. The recurrent neural network can capture the speed dynamics (such as higher order gradients) of the machine control point sequences via recurrent connections, which, by one approach, are unrolled across time steps (or sequence steps) with the same underlying parameters applied at each step.

While standard connections can be applied synchronously to propagate each layer's activations to a subsequent layer at the same time step, the recurrent connections can be dynamic, passing information across adjacent time steps. This approach can enable the model to take into account the higher derivates of the mechanical axes in the preceding and upcoming control points while estimating the current machine control point parameters.

Further details that comport with these teachings will now be presented. It will be understood that the specific details of these examples are intended to serve an illustrative purpose and are not intended to suggest any particular limitations with respect to these teachings.

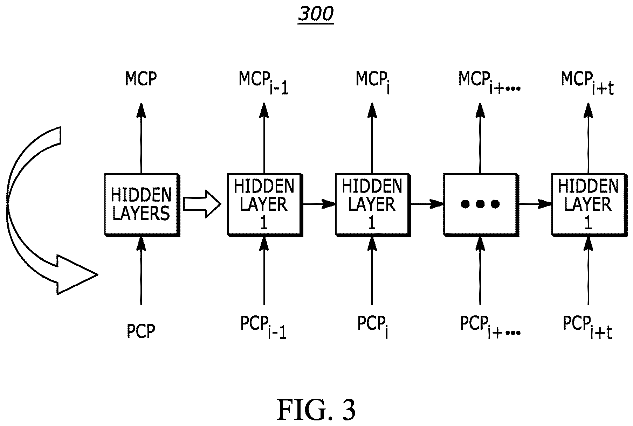

FIG. 3 presents a schematic representation of a recurrent neural network 300 configured in accordance with these teachings. Recurrent neural networks can be thought of as feedforward neural networks where each layer's parameters (both conventional and recurrent) are shared across time steps. Training such a model in this example includes collecting real machine control point parameters from the logs of the radiation treatment platform after each treatment. The accumulated parameters may represent anywhere from a few to many hundreds of treatments by the same platform.

These parameters are then served as the benchmark (labels) in supervised training of a model. The inputs to the recurrent neural network include (or, if desired, are exclusively) the optimized planning control point parameters (PCP).

The resultant trained model can be utilized to accurately estimate the machine control point (MCP) parameters well in advance of administering the treatment to a particular patient. The correctness of the parameter estimations can be verified based on the real time position and speed of the moving axes at the treating platform. In the case of detecting discrepancies, the estimated parameters can be swapped for the corresponding real time values to prevent the propagation of the errors to the upcoming machine control point estimations.

It will be appreciated that these teachings can also be applied in a situation where multiple similar radiation treatment platforms are tuned similarly so that they could be considered a same dosimetrically-equivalent machine group. The recurrent neural network model could be trained to satisfy the restrictions of the least capable machine in such a group so that the treatment time estimates are valid for all machines in the group.

So configured, these teachings can enable more accurate timing of the treatment plans since the evaluation is based on realistic machine trajectories taking into account the actual performance of the machine. Another advantage is that the recurrent neural network tends to provide more accurate estimation of the machine control point parameters as compared to current approaches, due at least in part to the fact that the recurrent neural network can capture the speed dynamics (i.e., higher order gradients) of the machine control point sequences via recurrent connections. This may allow, for example, the radiation treatment platforms to be controlled using the machine control points generated by the trained neural network.

These teachings will also accommodate combining the described model-based machine control point generation with more traditional methods to achieve improved performance. For example, one could use the slower of the two approaches and/or there could be a maximum allowed difference between the two methods.

Further aspects of the invention are provided by the subject matter of the following clauses (where it will be understood that any of these clauses can be combined with any one of more of the other clauses as appropriate):
Clause 1. A method comprising: by a control circuit: accessing information comprising physical settings for a particular radiation treatment platform to form a training corpus; training a neural network using the training corpus to provide a trained neural network.
Clause 2. The method of clause 1 wherein the physical settings comprise machine control points.
Clause 3. The method of clause 2 wherein the machine control points include at least two of a gantry angle, a multi-leaf collimator position, a dose rate, and a patient support position.
Clause 4. The method of clause 1 wherein training the neural network comprises training the neural network as a function of at least one of a passage of time, therapeutic uses of the particular radiation treatment platform, and completion of at least one maintenance activity.
Clause 5. The method of clause 1 wherein the training corpus does not include any information that pertains to any radiation treatment platform other than the particular radiation treatment platform.
Clause 6. The method of clause 1 wherein the neural network comprises a recurrent neural network.
Clause 7. The method of clause 1 wherein training the neural network comprises supervised training of the neural network.
Clause 8. The method of clause 1 further comprising: optimizing a radiation treatment plan for a particular patient using the particular radiation treatment platform, wherein the optimizing comprises optimizing plan control points to provide optimized plan control points; mapping the optimized plan control points to corresponding machine control points as a function, at least in part, of the trained neural network.
Clause 9. The method of clause 1 further comprising: outputting estimated machine control points from the trained neural network in advance of administering therapeutic radiation to a particular patient using the particular radiation treatment platform; during administration of the therapeutic radiation to the particular patient, comparing the estimated machine control points to real-time physical parameter values of the particular radiation treatment platform and substituting at least some real-time physical parameter values for corresponding ones of the estimated machine control points upon detecting a triggering discrepancy while continuing administration of the therapeutic radiation to the particular patient.
Clause 10. An apparatus comprising: a control circuit configured as a neural network that has been trained by a training corpus that comprises physical settings for a particular radiation treatment platform.
Clause 11. The apparatus of clause 10 wherein the physical settings comprise machine control points.
Clause 12. The apparatus of clause 11 wherein the machine control points include at least two of a gantry angle, a multi-leaf collimator position, a dose rate, and a patient support position.
Clause 13. The apparatus of clause 10 wherein the neural network was trained repeatedly as a function of at least one of a passage of time, therapeutic uses of the particular radiation treatment platform, and completion of at least one maintenance activity.
Clause 14. The apparatus of clause 10 wherein the training corpus does not include any information that pertains to any radiation treatment platform other than the particular radiation treatment platform.
Clause 15. The apparatus of clause 10 wherein the neural network comprises a recurrent neural network.
Clause 16. The apparatus of clause 10 wherein the neural network was trained via supervised training.
Clause 17. The apparatus of clause 10 wherein the control circuit is further configured to: optimize a radiation treatment plan for a particular patient using the particular radiation treatment platform, wherein the optimizing comprises optimizing plan control points to provide optimized plan control points; map the optimized plan control points to corresponding machine control points as a function, at least in part, of the neural network.
Clause 18. The apparatus of clause 10 wherein the control circuit is further configured to: output estimated machine control points from the neural network in advance of administering therapeutic radiation to a particular patient using the particular radiation treatment platform; during administration of the therapeutic radiation to the particular patient, compare the estimated machine control points to real-time physical parameter values of the particular radiation treatment platform and substitute at least some real-time physical parameter values for corresponding ones of the estimated machine control points upon detecting a triggering discrepancy while continuing administration of the therapeutic radiation to the particular patient.
Clause 19. A method comprising: optimizing a radiation treatment plan for a particular patient using a particular radiation treatment platform, wherein the optimizing comprises optimizing plan control points to provide optimized plan control points; mapping the optimized plan control points to corresponding machine control points as a function, at least in part, of a trained neural network that was trained with a training corpus that comprised previous physical settings for the particular radiation treatment platform.
Clause 20. The method of clause 19 wherein the previous physical settings comprise previous machine control points.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method comprising:
by a control circuit:
accessing information comprising physical settings for a particular radiation treatment platform to form a training corpus;
training a neural network using the training corpus to provide a trained neural network.

2. The method of claim 1 wherein training the neural network comprises training the neural network as a function of at least one of a passage of time, therapeutic uses of the particular radiation treatment platform, and completion of at least one maintenance activity.

3. The method of claim 1 or 2 further comprising:
optimizing a radiation treatment plan for a particular patient using the particular radiation treatment platform, wherein the optimizing comprises optimizing plan control points to provide optimized plan control points;
mapping the optimized plan control points to corresponding machine control points as a function, at least in part, of the trained neural network.

4. The method of claim 1, 2 or 3 further comprising:
outputting estimated machine control points from the trained neural network in advance of administering therapeutic radiation to a particular patient using the particular radiation treatment platform;
during administration of the therapeutic radiation to the particular patient, comparing the estimated machine control points to real-time physical parameter values of the particular radiation treatment platform and substituting at least some real-time physical parameter values for corresponding ones of the estimated machine control points upon detecting a triggering discrepancy while continuing administration of the therapeutic radiation to the particular patient.

5. An apparatus comprising:
a control circuit configured as a neural network that has been trained by a training corpus that comprises physical settings for a particular radiation treatment platform.

6. The method of claim 1, 2, 3 or 4 or the apparatus of claim 5 wherein the physical settings comprise machine control points.

7. The method or apparatus of claim 6 wherein the machine control points include at least two of a gantry angle, a multi-leaf collimator position, a dose rate, and a patient support position.

8. The apparatus of claim 5, 6 or 7 wherein the neural network was trained repeatedly as a function of at least one of a passage of time, therapeutic uses of the particular radiation treatment platform, and completion of at least one maintenance activity.

9. The method of claim 1, 2, 3, 4, 6 or 7, or the apparatus of claim any one of claims 5 to 8 wherein the training corpus does not include any information that pertains to any radiation treatment platform other than the particular radiation treatment platform.

10. The method of claim 1, 2, 3, 4, 6, 7 or 9, or the apparatus of any one of claims 5 to 9 wherein the neural network comprises a recurrent neural network.

11. The method of claim 1, 2, 3, 4, 6, 7, 9 or 10, or the apparatus of claim any one of claims 5 to 10 wherein the neural network is or was trained via supervised training.

12. The apparatus of any one of claims 5 to 11 wherein the control circuit is further configured to:
optimize a radiation treatment plan for a particular patient using the particular radiation treatment platform, wherein the optimizing comprises optimizing plan control points to provide optimized plan control points;
map the optimized plan control points to corresponding machine control points as a function, at least in part, of the neural network.

13. The apparatus of any one of claims 5 to 12 wherein the control circuit is further configured to:
output estimated machine control points from the neural network in advance of administering therapeutic radiation to a particular patient using the particular radiation treatment platform;
during administration of the therapeutic radiation to the particular patient, compare the estimated machine control points to real-time physical parameter values of the particular radiation treatment platform and substitute at least some real-time physical parameter values for corresponding ones of the estimated machine control points upon detecting a triggering discrepancy while continuing administration of the therapeutic radiation to the particular patient.

14. A method comprising:
optimizing a radiation treatment plan for a particular patient using a particular radiation treatment platform, wherein the optimizing comprises optimizing plan control points to provide optimized plan control points;
mapping the optimized plan control points to corresponding machine control points as a function, at least in part, of a trained neural network that was trained with a training corpus that comprised previous physical settings for the particular radiation treatment platform.

15. The method of claim 14 wherein the previous physical settings comprise previous machine control points.
